# EUROPÄISCHE PATENTSCHRIFT

(11) **EP 3 298 980 B1**
(45) Veröffentlichungstag und Bekanntmachung des Hinweises auf die Patenterteilung: **12.12.2018**
(21) Anmeldenummer: 17177375.7
(22) Anmeldetag: 22.06.2017
(51) Int. Cl.: A61B 46/10, A61B 17/02

(54) **ÜBERZUG FÜR CHIRURGISCHES INSTRUMENT**
DRAPE FOR SURGICAL INSTRUMENT
COUVERTURE POUR INSTRUMENT CHIRURGICAL

(30) Priorität: 26.05.2016 DE 202016102813 U
(43) Veröffentlichungstag der Anmeldung: 28.03.2018
(73) Patentinhaber: Lode, Jolanta, 24232 Schönkirchen (DE)
(72) Erfinder: Lode, Jolanta, 24232 Schönkirchen (DE)
(74) Vertreter: Lobemeier, Martin Landolf

(56) Entgegenhaltungen:
- WO-A1-2013/036625
- US-A- 4 834 090
- US-A1- 2004 249 473
- US-A1- 2015 320 411

## Beschreibung

Die Erfindung betrifft einen Überzug für ein chirurgisches Instrument.

Überzüge für chirurgische Instrumente sind beispielsweise aus der DE 20 2007 008 070 U1, der DE 10 2009 023 205 A1 und der DE 10 2008 017 898 A1 bekannt.

Wenngleich sich diese Überzüge bei Operationen als sehr vorteilhaft erwiesen haben, ist die Vorbereitung zur Operation arbeits- und zeitaufwändig.

Außerdem stellt sich das Problem, dass für den Bezug der beiden Schenkel einer Operationsklemme zwei Überzüge zu verwenden sind. Dieses Vorgehen ist nicht nur sehr zeit- und arbeitsaufwändig. Darüber hinaus wird die Funktion der Operationsklemme aufgrund des hohen durch zwei Überzüge anfallenden Materialaufwands beeinträchtigt.

Wenngleich zu diesem Zweck auch besonders ausgestaltete Überzüge, wie beispielsweise aus der US 2015/0320411 A1 und der US 4 834 090 A bekannt, verwendet werden können, erscheinen diese nur unzureichend an der Operationsklemme gesichert.

Aufgabe der Erfindung ist es daher, einen Überzug für ein chirurgisches Instrument, insbesondere für eine Operationsklemme zu schaffen, der schnell und einfach am chirurgischen Instrument zu befestigen ist und die Funktion des chirurgischen Instruments nicht behindert.

Diese Aufgabe wird erfindungsgemäß durch den Überzug mit den Merkmalen von Anspruch 1 gelöst. Die Unteransprüche geben vorteilhafte Ausgestaltungen der Erfindung wieder.

Der Grundgedanke der Erfindung ist es, an einem schlauchförmigen Abschnitt zur Aufnahme eins chirurgischen Instruments zusätzlich einen zweiten und dritten Abschnitt ebenfalls schlauchförmig auszubilden, wobei die Öffnungen dieser Abschnitte als Widerlager für das OP-Personal beim Beziehen der Operationsklemme verwendet werden können. Nach dem Beziehen eines chirurgischen Instruments können der zweite und dritte Abschnitt durch Verknoten oder Kleben mit der Operationsklemme verbunden und vor einem Verrutschen gesichert werden.

Erfindungsgemäß ist also ein Überzug für ein chirurgisches Instrument mit einem schlauchartigen ersten Abschnitt mit einer Öffnung zur Aufnahme eines chirurgischen Instruments, einem sich von einer Seite der Öffnung des ersten Abschnitts erstreckenden schlauchartigen zweiten Abschnitt, und einem sich von der anderen Seite der Öffnung des ersten Abschnitts erstreckenden schlauchartigen dritten Abschnitt vorgesehen, wobei der zweite und dritte Abschnitt jeweils eine Öffnung aufweisen, die der Öffnung des ersten Abschnitts entgegengesetzt auf der Außenseite des ersten Abschnitts angeordnet sind, wobei die Innenwandung des ersten Abschnitts in die Außenwandung des zweiten und dritten Abschnitts übergeht, wohingegen die Außenwandung des ersten Abschnitts in die Innenwandung des zweiten und dritten Abschnitts übergeht.

Bevorzugt ist der erste Abschnitt aus zwei schlauchartigen Abschnitten zur Aufnahme von zwei Abschnitten eines chirurgischen Instruments aufweist. Insbesondere sind die beiden Abschnitte zur Aufnahme der Schenkel einer Operationsklemme ausgebildet. Besonders bevorzugt bilden die beiden schlauchartigen Abschnitte einen eine gemeinsame Öffnung aufweisenden Abschnitt aus, sodass ein einfaches Überziehen der beiden Schenkel der Operationsklemme ermöglicht ist.

Bevorzugt ist der erste schlauchartige Abschnitt blind endend ausgebildet. Ebenso können der zweite und der dritte Abschnitt blind endend ausgebildet sein.

Eine vorteilhafte Ausgestaltung des Überzugs ergibt sich, wenn die Öffnungen des zweiten und dritten schlauchförmigen Abschnitts V-förmig ausgebildet sind, da in diesem Fall die Finger leichter in den Hohlraum des dritten und vierten Abschnitts gleiten können und darin ein Widerlager zum Überziehen finden können.

Aus hygienischen und fertigungstechnischen Aspekten, sowie aufgrund von Sicherheitsanforderungen ist es vorteilhaft, wenn der Überzug aus einer durchgehenden Textilbahn besteht. In diesem Fall sind der zweite und dritte Abschnitt durch Umschlagen und Vernähen eines Textilbahnabschnitts oder durch Vernähen und darauf folgendes Umstülpen des Textilbahnabschnitts gebildet.

Aus Sicherheitsgründen bei der Verwendung des Überzugs erscheint es vorteilhaft, wenn der Überzug einen Röntgenstreifen aufweist. Dieser ist insbesondere in den Überzug eingewebt.

Schließlich hat es sich bei der Handhabung des Überzugs als vorteilhaft erwiesen, wenn dieser aus Polyamid besteht.

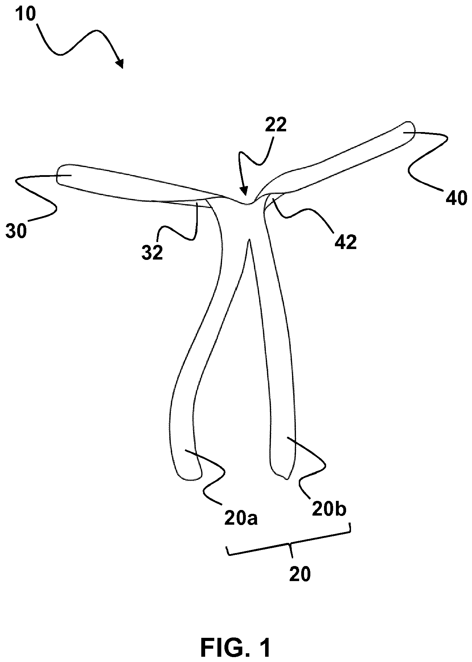

Die Erfindung wird im Folgenden anhand eines in der einzigen Fig. 1 dargestellten, besonders bevorzugt ausgestalteten Ausführungsbeispiels für den Überzug einer Operationsklemme näher erläutert, wobei Fig. 1 den schematischen Aufbau eines besonders ausgestalteten Ausführungsbeispiels nach der Erfindung zeigt:
Der Überzug 10 weist einen schlauchartigen ersten Abschnitt 20 mit einer Öffnung 22 zur Aufnahme der Schenkel einer Operationsklemme. Der schlauchartige Abschnitt 20 ist für diesen Zweck in zwei schlauchförmige Abschnitte 20a, 20b unterteilt.

Weiter ist ein sich auf einer Seite der Öffnung 22 des ersten Abschnitts 20 erstreckender schlauchartiger zweiter Abschnitt 30 und ein sich von der anderen Seite der Öffnung 22 des ersten Abschnitts 20 erstreckender schlauchartiger dritter Abschnitt 40 vorgesehen.

Fig. 1 ist zu entnehmen, dass der zweite und dritte Abschnitt 30, 40 jeweils eine V-förmige Öffnung 32, 42 aufweisen, die der Öffnung 22 des ersten Abschnitts 20 entgegengesetzt auf der Außenseite des ersten Abschnitts 20 angeordnet sind. Mit anderen Worten geht die Innenwandung des ersten Abschnitts 20 in die Außenwandung des zweiten und dritten Abschnitts 30, 40 über, wohingegen die Außenwandung des ersten Abschnitts 20 in die Innenwandung des zweiten und dritten Abschnitts übergeht.

Das Operationspersonal kann in außen am ersten Abschnitt angeordneten Öffnungen 32, 42 eingreifen, ohne mit der Operationsklemme zu kollidieren, woraus sich eine einfache Handhabung des dargestellten Ausführungsbeispiels ergibt.

## Patentansprüche

1. Überzug (10) für ein chirurgisches Instrument mit
- einem schlauchartigen ersten Abschnitt (20) mit einer Öffnung (22) zur Aufnahme eines chirurgischen Instruments, und
- einem sich von einer Seite der Öffnung (22) des ersten Abschnitts (20) erstreckenden schlauchartigen zweiten Abschnitt (30),
**gekennzeichnet durch**
- einen sich von der anderen Seite der Öffnung (22) des ersten Abschnitts (20) erstreckenden schlauchartigen dritten Abschnitt (40),
wobei der zweite und dritte Abschnitt (30, 40) jeweils eine Öffnung (32, 42) aufweisen, die der Öffnung (22) des ersten Abschnitts (20) entgegengesetzt auf der Außenseite des ersten Abschnitts (20) angeordnet sind,
wobei die Innenwandung des ersten Abschnitts (20) in die Außenwandung des zweiten und dritten Abschnitts (30, 40) übergeht, wohingegen die Außenwandung des ersten Abschnitts (20) in die Innenwandung des zweiten und dritten Abschnitts (30,40) übergeht.

2. Überzug (10) nach Anspruch 1, **dadurch gekennzeichnet, dass** der erste Abschnitt (20) aus zwei schlauchartigen Abschnitten (20a, 20b) zur Aufnahme von zwei Abschnitten eines chirurgischen Instruments aufweist.

3. Überzug (10) nach Anspruch 2, **dadurch gekennzeichnet, dass** die beiden schlauchartigen Abschnitte (20a, 20b) einen eine gemeinsame Öffnung aufweisenden Abschnitt ausbilden.

4. Überzug (10) nach einem der vorhergehenden Ansprüche, **dadurch gekennzeichnet, dass** der erste schlauchartige Abschnitt (20) blind endend ausgebildet ist.

5. Überzug (10) nach einem der vorhergehenden Ansprüche, **dadurch gekennzeichnet, dass** die Öffnungen des zweiten und dritten schlauchförmigen Abschnitts (30, 40) V-förmig ausgebildet sind.

6. Überzug (10) nach einem der vorhergehenden Ansprüche, **dadurch gekennzeichnet, dass** der Überzug (10) aus einer durchgehenden Textilbahn besteht.

7. Überzug (10) nach Anspruch 6, **dadurch gekennzeichnet, dass** der zweite und dritte Abschnitt (30, 40) durch Umschlagen und Vernähen eines Textilbahnabschnitts oder durch Vernähen und Umstülpen des Textilbahnabschnitts gebildet sind.

8. Überzug (10) nach einem der vorhergehenden Ansprüche, **gekennzeichnet durch** einen Röntgenstreifen.

9. Überzug (10) nach Anspruch 8, dass der Röntgenstreifen in den Überzug (10) eingewebt ist.

10. Überzug (10) nach einem der vorhergehenden Ansprüche, **dadurch gekennzeichnet, dass** der Überzug (10) aus Polyamid besteht.

## Claims

1. Drape (10) for a surgical instrument comprising
- a tubular first portion (20) comprising an opening (22) for receiving a surgical instrument, and
- a tubular second portion (30) extending from one side of the opening (22) of the first portion (20),
**characterized by**
- a tubular third portion (40) extending from the other side of the opening (22) of the first portion (20),
the second and third portions (30, 40) each comprising an opening (32, 42) arranged on the outer side of the first portion (20) opposite the opening (22) of the first portion (20),
the inner wall of the first portion (20) transitioning into the outer wall of the second and third portions (30, 40), while the outer wall of the first portion (20) transitions into the inner wall of the second and third portion (30, 40).

2. Drape (10) according to claim 1, **characterized in that** the first portion (20) comprises two tubular portions (20a, 20b) for receiving two portions of a surgical instrument.

3. Drape (10) according to claim 2, **characterized in that** the two tubular portions (20a, 20b) form a portion comprising a common opening.

4. Drape (10) according to any of the preceding claims, **characterized in that** the first tubular portion (20) is formed having a blind end.

5. Drape (10) according to any of the preceding claims, **characterized in that** the openings of the second and third tubular portions (30, 40) are V-shaped.

6. Drape (10) according to any of the preceding claims, **characterized in that** the drape (10) consists of a continuous textile web.

7. Drape (10) according to claim 6, **characterized in that** the second and third portions (30, 40) are formed by turning up and sewing up a textile web portion or by sewing up and turning the textile web portion inside out.

8. Drape (10) according to any of the preceding claims, **characterized by** a strip of X-ray film.

9. Drape (10) according to claim 8, that the strip of X-ray film is woven into the drape (10).

10. Drape (10) according to any of the preceding claims, **characterized in that** the drape (10) consists of polyamide.

## Revendications

1. Housse (10) destinée à un instrument chirurgical avec
- une première partie (20) de forme tubulaire dotée d'un orifice (22) pour l'admission de l'instrument chirurgical, et
- une deuxième partie (30) de forme tubulaire s'étendant à partir d'un côté de l'orifice (22) de la première partie (20),
**caractérisée par**
- une troisième partie (40) de forme tubulaire s'étendant à partir de l'autre côté de l'orifice (22) de la première partie (20),
où les deuxième et troisième parties (30, 40) présentent respectivement des orifices (32, 42) qui sont disposés opposés à l'orifice (22) de la première partie (20) sur le côté extérieur de la première partie (20),
où la paroi intérieure de la première partie (20) se fond dans la paroi extérieure de la deuxième et de la troisième partie (30, 40), la paroi extérieure de la première partie (20) confondant par contre avec la paroi intérieure des deuxième et troisième parties (30, 40).

2. Housse (10) selon la revendication 1, **caractérisée en ce que** la première partie (20) présente deux parties de forme tubulaire (20a, 20b) destinées à la réception de deux parties d'un instrument chirurgical.

3. Housse (10) selon la revendication 2, **caractérisée en ce que** les deux parties de forme tubulaire (20a, 20b) forment une partie présentant un orifice commun.

4. Housse (10) selon l'une des revendications précédentes, **caractérisée en ce que** la première partie de forme tubulaire (20) est conçue avec une extrémité aveugle.

5. Housse (10) selon l'une des revendications précédentes, **caractérisée en ce que** les orifices des deuxième et troisième parties (30, 40) sont conçus en forme de V.

6. Housse (10) selon l'une des revendications précédentes, **caractérisée en ce que** la housse (10) est constituée d'une bande de textile d'un seul tenant.

7. Housse (10) selon la revendication 6, **caractérisée en ce que** les deuxième et troisième parties (30, 40) sont formées par un repli et la couture d'une partie de la bande de textile ou par la couture et le rabattement de la partie de bande de textile.

8. Housse (10) selon l'une des revendications précédentes, **caractérisée par** un marqueur radio-opaque.

9. Housse (10) selon la revendication 8, **caractérisée en ce que** le marqueur radio-opaque est tissé dans la housse (10).

10. Housse (10) selon l'une des revendications précédentes, **caractérisée en ce que** la housse (10) est constituée de polyamide.
